# FASCICULE DE BREVET EUROPEEN

(11) **EP 1 041 082 B1**
(45) Date de publication et mention de la délivrance du brevet: **17.04.2002**
(21) Numéro de dépôt: 00109844.1
(22) Date de dépôt: 19.10.1995
(51) Int. Cl.: C07K 1/08

(54) **Procédé de synthése peptidique à partir de N-carboxyanhydrideaminoacides**
Verfahren zur Herstellung von Peptiden aus N-Carboxyanhydridaminosäuren
Process of peptidic synthesis starting from N-carboxyanhydride amino acids

(30) Priorité: 24.10.1994 FR 9412779
(43) Date de publication de la demande: 04.10.2000
(62) Demande divisionnaire de: 95934205.6
(73) Titulaire: Degussa AG, 40474 Düsseldorf (DE)
(72) Inventeur: Commeyras, Auguste, 34830 Clapiers (FR); Collet, Hélène, Le Jardin aux Fontaines, 34000 Montpellier (FR); Mion, Louis, 34080 Montpellier (FR); Benefice, Sylvie, 34080 Montpellier (FR); Calas, Patrick, 34000 Montpellier (FR); Choukroun, Henri, 34830 Clapiers (FR); Taillades, Jaques, 34170 Castelnau Le Lez (FR); Bied, Catherine, 34009 Montpellier (FR)

(56) Documents cités:
- CHEMICAL ABSTRACTS, vol. 92, no. 15, 14 avril 1980 (1980-04-14) Columbus, Ohio, US; abstract no. 129280v, V V LUTSENKO: "Acylation of alkyl esters of alpha-amino acids by nitrosohydantoic acid" page 741; XP002007190 & ZH. ORG. KHIM., vol. 15, no. 9, 1979, pages 1973-1976,
- CHEMICAL ABSTRACTS, vol. 75, no. 17, 25 octobre 1971 (1971-10-25) Columbus, Ohio, US; abstract no. 110012m, V V LUTSENKO ET AL.: "N-Nitrosoureides. II. Nitrosation of some alpha-ureido acids" page 410; XP002007191 & ZH. ORG. KHIM., vol. 7, no. 6, 1971, pages 1152-1159,
- R HIRSCHMANN ET AL.: "The controlled synthesis of peptides in aqueous medium. The preparation and use of novel alpha-amino acid N-Carboxyanhydride" JOURNAL OF THE AMERICAN CHEMICAL SOCIETY, vol. 93, no. 11, 2 juillet 1971 (1971-07-02), pages 2746-2754, XP002007186 DC US
- K INOUYE & K WATANABE: "Formation and degradation of urea derivatives in the azide method of peptide synthesis. Part 2. Acidolytic degradation of urea derivatives" JOURNAL OF THE CHEMICAL SOCIETY, PERKIN TRANSACTIONS 1, no. 17, 1977, pages 1911-1915, XP002007187 LETCHWORTH GB
- K INOUYE ET AL.: "Formationa nd degradation of urea derivatives in the azide method of peptide synthesis. Part 1. The Curtius rearrangement and urea formation" JOURNAL OF THE CHEMICAL SOCIETY, PERKIN TRANSACTIONS 1, no. 17, 1977, pages 1905-1911, XP002007188 LETCHWORTH GB
- S PAIK & E H WHITE: "N-Acyl-N-Nitrosoamino acids and peptides" TETRAHEDRON LETTERS, vol. 435, no. 42, 17 octobre 1994 (1994-10-17), pages 7731-7734, XP002007189 OXFORD GB
- CHEMICAL ABSTRACTS, vol. 101, no. 15, 8 octobre 1984 (1984-10-08) Columbus, Ohio, US; abstract no. 125184y, M SAKURAI & H YANAGAWA: "Prebiotic synthesis of amino acids from formaldehyde and hydroxylamine in a modified sea medium" page 258; XP002007192 & ORIGINS LIFE, vol. 14, no. 1-4, 1984, pages 171-176,
- CHEMICAL ABSTRACTS, vol. 84, no. 17, 26 avril 1976 (1976-04-26) Columbus, Ohio, US; abstract no. 122296f, I K ROMANOVSKAYA ET AL.: "Solid-phase synthesis of (1-asparagine, 5-valine, 8-alanine) and (1-hydantoic acid, 5-valine, 8-alanine) angiotensin II" page 588; XP002007193 & BIOORG. KHIM., vol. 1, no. 9, 1975, pages 1257-1262,
- R G DENKEWALTER ET AL. : "The controlled synthesis of peptides in aqueous medium. I. The use of alpha-amino acid N-Carboxyanhydrides" JOURNAL OF THE AMERICAN CHEMICAL SOCIETY., vol. 88, no. 13, 5 juillet 1966 (1966-07-05), pages 3163-3164, XP002140134 AMERICAN CHEMICAL SOCIETY, WASHINGTON, DC., US ISSN: 0002-7863

## Description

L'invention concerne un nouveau procédé de synthèse peptidique à partir de N-carboxyanhydrideaminoacides.

Les peptides sont des molécules biologiquement actives d'un intérêt considérable et leur synthèse a été étudiée depuis fort longtemps, cette synthèse pouvant s'effectuer par des méthodologies diverses, aussi bien en phase homogène qu'en phase solide ou supportée. Dans un cas comme dans l'autre, ces méthodologies utilisent des acides aminés protégés sur une fonction, créent ou allongent le peptide sur la fonction libre restante au moyen d'un acide aminé bloqué, débloquent cette dernière fonction et poursuivent ainsi pas à pas la construction de la chaîne. Pour avoir un peptide libre les fonctions terminales doivent évidemment être débloquées. Le solvant utilisé est essentiellement un solvant organique. De telles méthodologies tout en étant efficaces sont néanmoins très lourdes.

L'invention a pour objectif la mise au point d'un procédé de synthèse peptidique tolérant l'utilisation d'acides aminés non protégés et conduisant directement à un peptide libre.

A cet effet, le procédé conforme à l'invention pour la synthèse de peptides se caractérise en ce que
(a) on prépare un N-carboxyanhydrideaminoacide de formule : 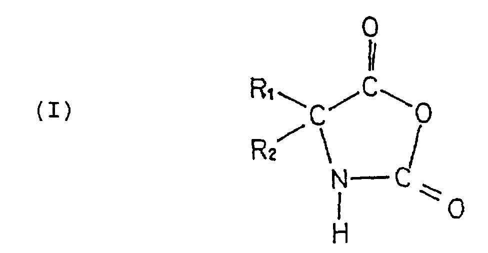 où R₁ et R₂ sont un hydrogène ou un radical alkyl, cycloalkyl, aryl ou aryl alkyl, substitué ou non par une ou des fonctions alcool, thiol, amine, sulfure, acide ou amide par décomposition en N₂ R'OH et N-carboxyanhydrideaminoacide d'un N-[N'-nitroso-N'-(R')carbamoyl]aminoacide de formule : 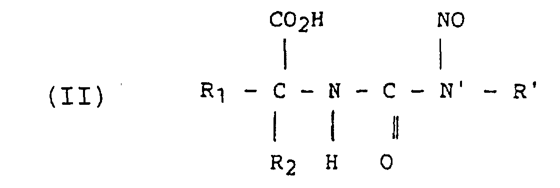 où R' est un radical du groupe H, CH₃, CH₂CH₂Cl.
(b) on réalise une réaction d'addition entre le composé (I) et un acide aminé ou un peptide ayant au moins une fonction (α-aminé libre, en vue d'obtenir un dipeptide ou un peptide de rang supérieur par rapport au peptide additionné.

Il est à noter que la publication J. Am. Chem. Soc. 88(13), pages 3613-3614 (1066) divulguait déjà un procédè de préparation des peptides à partir d'acides aminés et de N-carboxyanhydrides d'acides aminés.

Cependant, vis-à-vis de ce procédè antérieur, le procédè selon l'invention présente l'avantage de tolérer l'utilisation d'acides aminés non protégés et de conduire directement à un peptide libre.

Les inventeurs ont en effet mis en évidence que, de façon inattendue, l'on peut obtenir directement des peptides libres, c'est-à-dire ayant leur fonction aminée non protégée, par action des N-(N'-nitrosocarbamoyl)aminoacides (II) sur un acide aminé ou un peptide ayant leur fonction α-aminé et carboxylique libre.

Le procédé de l'invention permet de créer un dipeptide libre ou d'allonger d'une unité le peptide initial. La liaison peptidique formée est celle liant la fonction amine de l'acide aminé (ou du peptide) présent dans le milieu avec le groupement carboxyle du composé (I).

Dans le cas où la fonction carboxylique de l'acide aminé (ou du peptide à n fragments d'amino acides), présent dans la solution ou ajouté à celle-ci, est bloqué, le dipeptide ou le peptide à (n + 1 ) acides aminés formé, peut conserver le blocage de cette fonction.

Selon un mode de mise en oeuvre préféré, on choisit comme composé de départ un composé (II) dans lequel R' est un hydrogène, en vue d'obtenir, au terme de la décomposition, de l'eau comme sous-produit de décomposition. Cette eau peut demeurer associée au peptide formée sans inconvénient et sans nécessité d'une séparation.

On peut choisir comme radical R1 un résidu d'acide aminé naturel et comme radical R2 l'hydrogène en vue de synthétiser des peptides naturels ; on peut également choisir comme radical R1 un résidu d'acide aminé modifié et comme radical R2 un groupement méthyl, éthyl, propyl, pour obtenir un peptide modifié. Comme cela est connu en soi, cette modification permet de moduler les propriétés des peptides en fonction des applications.

Le procédé de l'invention est avantageusement mis en oeuvre selon les deux modes opératoires décrits ci-après.

En premier lieu, la réaction de décomposition peut être réalisée dans un solvant organique anhydre. Dans ce cas, la réaction d'addition consiste à ajouter dans le milieu réactif un acide aminé ou un peptide préparé préalablement. Les expérimentations semblent montrer que ce mode opératoire conduit à des peptides ce masse moléculaire élevée.

Un autre mode opératoire consiste à opérer la décomposition en milieu aquo-organique à un pH sensiblement compris entre 6 et 10. La masse moléculaire des peptides obtenus est dans ce cas significativement plus basse. Il est possible pour réaliser l'addition d'ajouter dans le milieu aquo-organique un acide aminé ou un peptide préparé préalablement. Cette mise en oeuvre, lorsqu'elle est réalisée dans un milieu aquo-organique tamponnée au moyen de bicarbonate alcalin (pH compris entre 6,5 et 7,5), permet d'ajouter un seul amino acide (provenant de la décomposition) sur l'acide aminé ou le peptide introduit dans le milieu. Une telle technique "pas à pas" offre la possibilité de synthétiser un hétéropeptide désiré par synthèses répétées.

En milieu aquo-organique, il est également possible, sans introduction d'acides aminés ou peptides dans le milieu, de réaliser une addition sur le N-carboxyanhydride d'un acide aminé provenant d'une hydrolyse partielle de ce N-carboxyanhydride. On obtient dans ce cas un homopeptide dont la masse molaire est d'autant plus grande que le pourcentage en eau du milieu est plus faible.

Les conditions de température de la décomposition et de l'addition ne paraissent pas critiques en pratique, on peut opérer à température ambiante toutefois, dans certains cas, une température plus élevée peut être choisie pour la décomposition afin de la favoriser (50°C par exemple), cependant qu'une température plus basse (de l'ordre de - 10°C) réduit dans la réaction d'addition les risques de réactions parasites.

Il est à noter que la synthèse des N-(N'nitrosocarbamoyl)aminoacides (II) est décrite dans la littérature (DE-OS 2,615,594, Japan Kokai 78 103,441, EPA 88 105 584 2 pour R' = H et Bul Chem Soc Japan, vol. 48 (4), 1333-1334 (1975), Arch. Pharm. Weinheim 314,910-917 (1981), 317,481-487 (1984), 322,863-872 (1989), pour R' = CH3, ClCH2CH2-... ). Ils sont décrits dans le cas où R' = H comme se décomposant en milieu acide à des pH inférieurs à 4 en donnant l'acide aminé correspondant, de l'azote et de l'anhydride carbonique (voir EPA 88 105 584 2). Ils sont jusqu'à présent utilisés pour hydrolyser la fonction carbamoyle. Les dérivés substitués (R' = CH₃, ClCH₂CH₂-... ) font quant à eux partie de la famille des nitrosourées N'-alkylées. Leurs propriétés antitumorales ont été étudiées, elles résulteraient de leur pouvoir alkylant (Arch Pharm 322,863-872 (1989)).

Selon une caractéristique du procédé de l'invention, les composés (II) peuvent être synthétisés directement dans le milieu réactionnel anhydre (dioxane, acétonitrile, ... ), aqueux à pH inférieur à 4 ou aquo-organique à pH inférieur à 4 par action d'un agent nitrosant tel que NO, NO₂H, N₂O₃, N₂O₄, ... utilisé pur ou en mélange avec un agent oxydant (oxygène, ou tout autre agent tel que Fe3', Cu2+, couples métalliques, ou oxydation électrochimique) sur un N-[N'-(R')carbamoyl]aminoacide de formule :

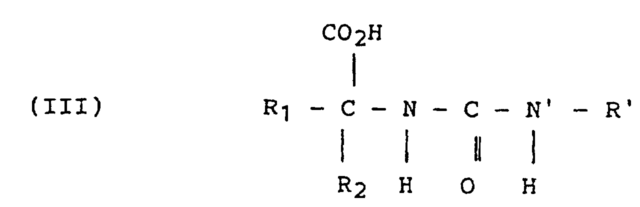

Un intermédiaire réactionnel dans cette réaction a été déterminé comme étant le composé N-nitrosoN-carbamoylaminoacide de formule (IV) qui, dans des conditions opératoires différentes de celles utilisées dans ce procédé, peut se décomposer en partie pour donner un ahydroxyacide :

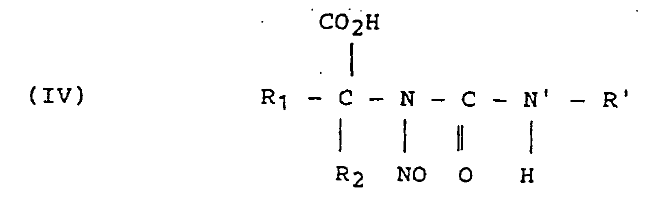

Les composés (II) peuvent également être préparés in situ dans le milieu réactionnel en deux étapes : en faisant d'abord réagir à un pH inférieur à 8 de l'acide isocyanique (HNCO), un sel de cet acide ou un isocyanate de méthyle ou de chloroéthyle sur un aminoacide pour obtenir un N-[N'-(R')carbamoyl]aminoacide de formule : 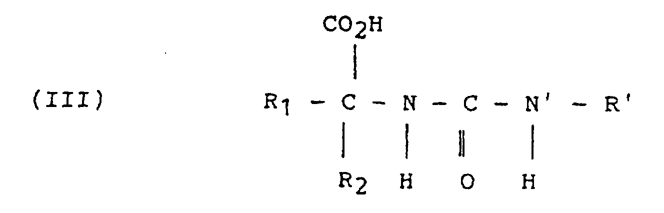 et en faisant ensuite réagir un agent nitrosant sur le N-[N'-(R)carbamoyl]aminoacide formé.

Il faut souligner que les aminoacides utilisés (composés ajoutés ou composes de départ) peuvent être de chiralité D ou L, ou racémiques. Lorsque les composés utilisés sont énantiomeriquement purs, ils ne se racémisent pas au cours de la réaction.

La figure unique du dessin illustre le mécanisme de la synthèse peptidique visée par l'invention dans le cas particulier où R2 = H et R' = H. Selon ce mécanisme quel que soit son degré de substitution, le composé (II) se décompose en libérant de l'azote, de l'eau (ou de l'alcool selon la nature de R') et en donnant un N-carboxyanhydride (NCA) (I), via un α-isocyanatoacide (VI) selon l'hypothèse formulée sur la figure unique. Malgré leur sensibilité à l'hydrolyse, les (NCA) (I) réagissent concurremment en milieu aqueux (et bien entendu en milieu anhydre) sur les fonctions aminées libres (c'est-à-dire non protonés) des acides aminés (V) en donnant des peptides (VII, VIII, etc.) dont la fonction aminée terminale perd spontanément l'anhydride carbonique.

Le procédé de l'invention est illustré ci-après au moyen d'exemples de mise en oeuvre.

### EXEMPLE 1 : Synthèse du N-(N'-ni trosocarbamoyl)valine (compose II dans lequel R₁ = iPr, R₂ = H, R' = H) à partir de N-carbamoylvaline (compose III avec les radicaux précités)

1 mmole (160 mg) de N-carbamoylvaline racémique est placée sous agitation dans 20 ml de dioxane anhydre à 20°C. La solution est balayée pendant 1/2 heure par un courant d'azote. A cette solution est alors ajouté un mélange gazeux de 66 ml de NO et 26 ml d'air (70 % de NO). Après 1/4 d'heure de réaction, le solvant et l'excédent d'agent nitrosant sont chassés sous pression réduite conduisant à une masse incolore (190 mg) qui cristallise. La solution est analysée en HPLC (chromatographie liquide haute pression), sur une colonne C18 avec comme éluant une solution eau/acétonitrile 9/1 contenant 0,05 % CF3COOH et détectée en UV à 220 nm. L'analyse isocratique à 1 ml/mn donne deux signaux a 22 et 28 mn d'intensité relative 30/70. Une nouvelle analyse du même milieu réactionnel 60 minutes après la précédente montre que l'intensité relative de ces deux signaux s'inverse 70/30. L'analyse en RMN 1H (résonance magnétique nucléaire du proton) de ces deux situations a permis d'établir que les produits correspondant à ces deux signaux sont respectivement, la N-(N'-nitrosocarbamoyl)-valine (II) RMN ¹H (DMSO-d6 + TMS) ; δ (ppm) : 0,85 (d, 3H, CH₃) ; 0,95 (d, 3H, CH₃) ; 2,1 (m, 1H, CH) ; 4,3 (dd, 1H, CH) ; 7,3 (s, 1H, NH) 9,15 (s, 1H, NH) et la N-nitroso-N carbamoylvaline (IV) RMN ¹H (DMSO-d6 + TMS) ; δ (ppm) = 0,85 (d, 3H, CH₃) ; 0,95 (d, 3H, CH₃) ; 2,1 (m, 1H, CH) ; 4,3 (dd, 1H, CH) ; 9,0 (s, 2H, NH₂).

Une troisième analyse 60 minutes après la précédente montre la disparition du signal à 28 mn au profit du signal à 22 mn correspondant ainsi à la formation quasi quantitative de la N-(N'-nitrosocarbamoyl)valine (II), même si dans ce dernier chromatogramme commence à apparaître avec une très faible intensité un signal à 17 mn que l'on analyse dans l'exemple 2 ci-dessous.

### EXEMPLE 2 : Synthèse du N-carboxyanhydrideva-line (I ; R1 = iPr, R2 = H) à partir de N-(N'-nitrosocarbamoyl)valine (II)

1 mmole de N-(N'nitrosocarbamoyl)valine (II) provenant de l'exemple précédent placée dans 20 ml de dioxane anhydre sous une atmosphère d'hélium est maintenue à 30°C pendant 30 minutes au cours desquelles on observe un dégagement gazeux au sein de la solution. Ce dégagement est analysé en spectroscopie de masse comme étant de l'azote moléculaire (Masse 28). L'analyse de la solution en HPLC montre alors la présence d'un seul signal à 17 mn et la disparition du signal à 22 mn. Le milieu réactionnel est ensuite évaporé sous pression réduite de façon à éliminer le solvant et l'eau formée au cours de la réaction. Le produit brut (145 mg) cristallisé est identifié, en le comparant à un échantillon authentique, comme étant le N-carboyxanhydridevaline.

TF = 75°C produit brut, 81°C après recristallisation.

RMN ¹H (DMSO-d6 + TMS) ; δ (ppm) : 0,25 (d, 3H, CH₃) ; 0,95 (d, 3H, CH₃) ; 2,1 (m, 1H, CH) ; 9,15 (s, 1 H, NH).

### EXEMPLE 3 : Synthèse du N-carboxyanhydridevaline (I ; R1 = iPr, R2 = H) à partir de N-carbamoylvaline (III) dans un solvant organique

1 mmole de N-carbamoylvaline est placée dans 20 ml d'acétonitrile (contenant moins de 0,1 % d'eau) à température ambiante et sous atmosphère d'azote. Le mélange nitrosant (66 ml NO + 26 ml d'air) est ajouté. Après 30 minutes de réaction, l'excès d'agent nitrosant et le solvant sont chassés sous pression réduite. L'analyse de la solution en HPLC du produit brut cristallisé, (PF 74°C) montre la présence d'un seul signal à 17 mn caractéristique du N-carboxyanhydridevaline. Son spectre de RMN est conforme à celui donné dans l'exemple 2.

### EXEMPLE 4 : Synthèse du N-carboxyanhydridevaline (I ; R1 = iPr, R2 = H) à partir de N-carbamoylvaline (III) en milieu aquo-organique

1 mmole de N-carbamoylvaline est placée M dans 20 ml d'eau, à température ambiante sous atmosphère d'azote. Le mélange nitrosant (264 ml NO + 104 ml d'air) est ajouté. Après 30 minutes de réaction, l'excès d'agent nitrosant et le solvant sont chassés sous pression réduite. L'analyse de la solution en HPLC du produit brut, montre la présence du signal à 17 mn caractéristique du Ncarboxyanhydridevaline ainsi que du signal à 4,8 mn de la valine dans le rapport 80/20.

### EXEMPLE 5 : Synthèse du N-carboxyanhydridevaline (I ; R1 = iPr, R2 = H) à partir de la valine (V) en milieu aquo-organique

1 mmole de valine et 3 mmole de cyanate de potassium sont ajoutés à 20 ml d'eau, à température ambiante (pH égal à 4). La formation de N-carbamoylvaline -(III) est suivie en HPLC. Lorsque la formation de Ncarbamoylvaline (III) est complète (au bout de 2 heures), le milieu est placé sous atmosphère d'azote et le mélange nitrosant (264 ml NO + 104 ml d'air) est ajoute. Après 30 minutes de réaction, l'excès d'agent nitrosant et le solvant sont chassés sous pression réduite. L'analyse HPLC, montre comme dans l'exemple 4 la présence d'un signal caractéristique du N-carboxyanhydridevaline ainsi que d'un signal d'intensité plus faible de la Valine dans le rapport 80/20.

### EXEMPLE 6 : Synthèse du dipeptide Val-Val à partir du N-carboxyanhydridevaline (I)

1 mmole de D,L-N-carboxyanhydridevaline (I) préparé comme dans les exemples 2 ou 3, est placée dans 5 ml d'acétonitrile. Une solution aquo-organique (10 ml H₂O + 8 ml CH₃CN) contenant 1 mmole de sel de sodium de la D,L-valine et 2 mmoles de Na₂CO₃ est alors ajoutée à -10°C (pH = 7).

Après 30 minutes de réaction à -10°C, le milieu réactionnel est acidifié et la solution est analysée par HPLC : le dipeptide libre Val-Val est caractérisé par rapport à un échantillon authentique (HPLC 6,97 mn). Le produit brut obtenu est recristallisé et identifié par son spectre de masse M + 1 = 217. Le rendement est de 95 %.

### EXEMPLE 7 : Synthèse du dipeptide Val-Val à partir de D, L-N-(N'-nitrosocarbamoyl)valine (II)

1 mmole de D,L-N-(N'-nitrosocarbamoyl) valine (II) préparé comme dans l'exemple 1, est placée dans 5 ml d'acétonitrile à 30°C pendant 60 mn. Une solution aquo-organique (10 ml H20 + 8 ml CH3CN) contenant 1 mmole de sel de sodium de la D,L-valine et 2 mmoles de Na2CO3 est alors ajoutée à - 10°C (pH = 7).

La suite de la réaction est identique à l'exemple 6.

### EXEMPLE 8 : Synthèse du tripeptide Val-Ala-Phe à partir de N-(N'-nitrosocarbamoyl)valine

Selon le même mode opératoire que dans l'exemple 7, on additionne 1 mmole de sel de sodium de dipeptide Ala-Phe à la solution de N-(N'nitrosocarbamoyl)valine.

Le tripeptide Val-Ala-Phe est obtenu et identifié à partir d'un échantillon authentique en HPLC et spectroscopie de Masse. Le rendement en produit cristallisé est de 80 %.

### EXEMPLE 9 : Synthèse du dipeptide Ala-Phe à partir de N-[N'-nitroso-N'-(2-chloroéthyl)carbamoyl]alanine

Selon le même mode opératoire que dans l'exemple 7, on additionne 1 mmole de sel de sodium de la phenylalanine à la solution de N-[N', N'-(2-chloroéthyl)nitrosocarbamoyl]alanine (RMN ¹H (DMSO-d6 + TMS) ; δ (ppm) : 1,4 (d, 3H, CH3) ; 3,6 (t, 2H, CH2) ; 4,1 (t, 2H, CH2) ; 4,4 (q, 1H, CH) ; 8,9 (d, 1H, NH).

Le dipeptide Ala-Phe est obtenu et identifié à partir d'un échantillon authentique en HPLC et spectroscopie de Masse. Rendement 40 %.

### EXEMPLE 10 : Synthèse de la N-(N'nitrosocarbamoyl) alanine (II ; R1 = CH3, R2 = H, R' = H) puis sa décomposition en N-carboxyanhydridealanine (I)

1 mmole (132 mg) de D,L-N-carbamoylalanine (III) est mise en réaction comme dans l'exemple 1. L'analyse HPLC (éluant eau/acétonitrile 95/5 + 0,05 % CF₃CO₂H) montre comme pour la valine la formation d'une espèce sous contrôle cinétique à 15 mn évoluant ensuite vers une seconde espèce caractérisée par un signal 11 mn. Le spectre de RMN ¹H (DMSO-d6 + TMS) ; δ (ppm) 1,2 (d, 3H, CH₃) ; 4,05 (q, H, CH) ; 8 (s, 1H, NH) ; 8,95 (s, 1H, NH) caractérise la N-(N'-nitrosocarbamoyl)alanine. Cette dernière espèce évolue à son tour pour donner quantitativement le D,L-N-carboxyanhydridealanine caractérisé par son temps de rétention HPLC à 6,9 mn, son point de fusion (60°C) et son spectre de RMN.

### EXEMPLE 11 : Synthèse du D,L-N-(N'nitrosocarbamoyl)méthionine (II ; R1 = CH3S(CH2)2-, R2 = H, R' = H) puis sa décomposition en N-carboxyanhydride méthionine (I)

1 mmole de N-carbamoylméthionine (III) (192 mg) est placée dans 20 ml d'acétonitrile et traitée selon le mode opératoire de l'exemple 1. Après 5 minutes de réaction, l'excès d'agent nitrosant et le solvant sont éliminés sous pression réduite.

L'analyse de la solution en HPLC montre
- un pic de très faible intensité à 28 mn caractérisant la présence résiduelle de la N-nitroso-N-carbamoylméthionine (IV),
- un pic très intense à 22 mn caractérisant la N-(N'-nitrosocarbamoyl)méthionine (II) {(RMN ¹H (DMSO- d6 + TMS) ; δ (ppm) : 2,02 (m, 2H, CH₂) 2,06 (s, 3H, CH₃) ; 2,55 (t, 2H, CH₂) ; 4,58 (t, 1H, CH) ; 7,9 (s, 1H, NH) ; 8,2 (s, 1H, NH)},
- un pic également très intense à 17 mn caractérisant le N-carboxyanhydrideméthionine (II) {RMN ¹H (DMSO-d6 + TMS) ; δ (ppm) : 2,02 (m, 2H, CH₂) ; 2,06 (s, 3H, CH₃) ; 2,55 (t, 2H, CH2) ; 4, 6 (t, 1 H, CH) ; 9, 1 (s, 1H, NH)}.

Dans le cas de la méthionine, la décomposition totale du composé (II) en composé (I) nécessite un apport énergétique supérieur aux exemples précédents (50°C plusieurs heures).

L'hydrolyse du composé (I) ainsi obtenu conduit 93 % de méthionine et 7 % de méthionine sulfoxyde, montrant l'existence de possibilités de contrôle du pouvoir oxydant du milieu nitrosant utilisé.

### EXEMPLE 12 : Analyse de traces de N-carbamoylaminoacides (III) dans les peptides synthétisés.

La méthode HPLC précédemment utilisée permet de détecter les N-carbamoylaminoacides (III) a des concentrations aussi faibles que 10⁻⁶ moles par litre.

Les peptides synthétisés par cette méthode ont montré après cristallisation la présence de traces de N-carbamoylaminoacides (III) inférieures au ppm mais néanmoins visibles dans le bruit de fond du chromatogramme.

## Revendications

1. Procédé de synthèse peptidique,
**caractérisé en ce que**
(a) on prépare un N-carboxyanhydrideaminoacide de formule : 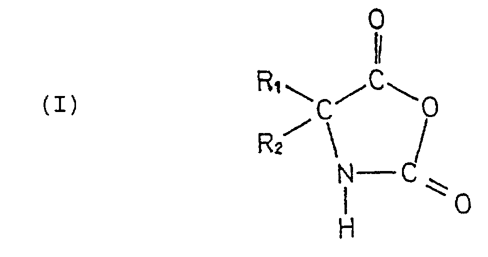 où R1 et R2 sont un hydrogène ou un radical alkyl, cycloalkyl, aryl ou aryl alkyl, substitué ou non par une ou des fonctions alcool, thiol, amine, sulfure, acide ou amide par décomposition en N2 R'OH et N-carboxyanhydrideaminoacide d'un N-[N'-nitroso-N'-(R')carbamoyl]aminoacide de formule : 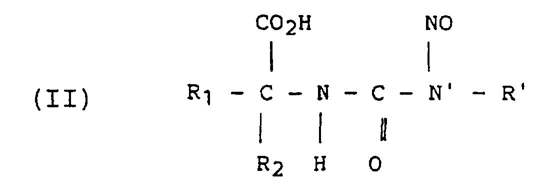 où R' est un radical du groupe H, CH₃, CH₂CH₂Cl.
(b) on réalise une réaction d'addition entre le composé (I) et un acide aminé ou un peptide ayant au moins une fonction (α-aminé libre, en vue d'obtenir un dipeptide ou un peptide de rang supérieur par rapport au peptide additionné.

2. Procédé selon la revendication 1,
**caractérisé en ce qu'**
on prépare le composé (II) dans lequel R' est un hydrogène, en vue d'obtenir, au terme de la décomposition, de l'eau comme sous-produit de décomposition.

3. Procédé selon l'une quelconque des revendications 1 à 2
**caractérisé en ce que**
on prépare un composé (I) dans lequel R₁ est un résidu d'acide aminé et R2 un hydrogène ou un groupement méthyl, éthyl ou propyl.

4. Procédé selon l'une des revendications 1 à 3,
**caractérisé en ce que**
la décomposition du composé (II) est réalisée dans un solvant organique anhydre, et la réaction d'addition consiste à ajouter dans le milieu réactif un acide aminé ou un peptide prépare préalablement.

5. Procédé selon l'une des revendications 1 à 3
**caractérisé en ce que**
la décomposition du composé (II) est réalisée en milieu aquo-organique de pH sensiblement compris entre 6 et 10, et la réaction d'addition consiste à ajouter dans le milieu réactif un acide aminé ou un peptide préparé préalablement.

6. Procédé selon la revendication 5,
**caractérisé en ce qu'**
on utilise pour opérer la décomposition et l'addition un milieu aquo-organique tamponné au moyen de bicarbonate alcalin à pH compris entre 6,5 et 7,5, en vue d'additionner un seul amino-acide (provenant de la décomposition) sur l'acide aminé ou le peptide introduit dans le milieu.

7. Procédé selon l'une des revendications 1 à 3
**caractérisé en ce que**
la décomposition du compose (II) est réalisée en milieu aquo-organique de pH sensiblement compris entre 6 et 10, et la réaction d'addition est réalisée entre le composé (I) et un acide aminé provenant d'une hydrolyse partielle du composé (I).

8. Procédé selon l'une des revendications 1 à 7,
dans lequel
(a) le N-[N'-nitroso-N'-(R' )carbamoyl]aminoacide est synthétisé directement dans le milieu réactionnel par action d'un agent nitrosant sur un N-[N'-(R')carbamoyl]aminoacide de formule :

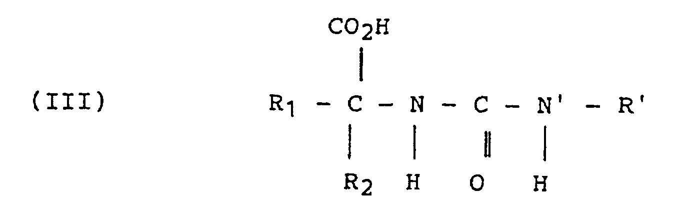

9. Procédé selon l'une des revendications 2 à 8,
dans lequel
le N-[N'-nitroso-N'(R')carbamoyl]aminoacide est synthétisé directement dans le milieu réactionnel en deux étapes :
. en faisant d'abord réagir de l'acide isocyanique, un sel de cet acide ou un isocyanate de méthyle ou de chloroéthyle sur un aminoacide pour obtenir un N-[N'-(R')carbamoyl]aminoacide de formule : 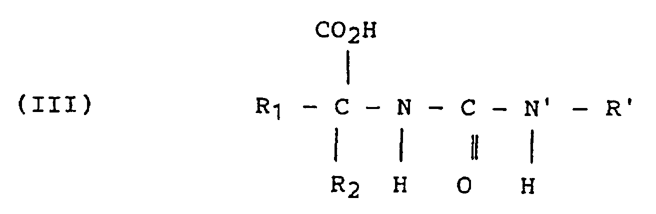
. et en faisant ensuite réagir un agent nitrosant sur le N-[N'-(R')carbamoyl}aminoacide formé.

## Claims

1. Process of peptide synthesis, **characterized in that**
(a) an N-carboxyanhydrideamino acid of formula: 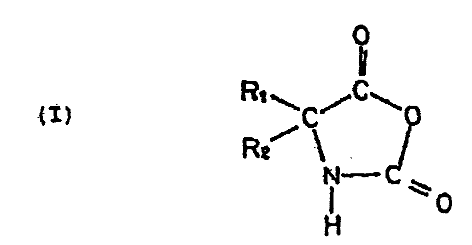 in which R₁ and R₂ are a hydrogen or an alkyl, cycloalkyl, aryl or arylalkyl radical, which may or may not be substituted with one or more alcohol, thiol, amine, sulphide, acid or amide functions, is prepared by decomposition to N₂ R'OH and N-carboxyanhydrideamino acid of an N-[N'-nitroso-N'-(R')carbamoyl]amino acid of formula: 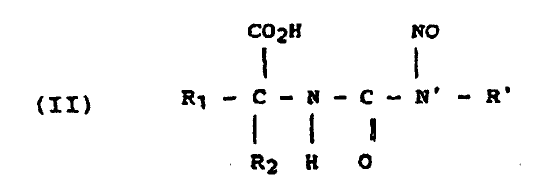 in which R' is a radical from the group H, CH₃ and CH₂CH₂Cl.
(b) an addition reaction is performed between compound (I) and an amino acid or a peptide containing at least one function (free α-amino, so as to obtain a dipeptide or a peptide which is higher in rank than the peptide added.

2. Process according to Claim 1, **characterized in that** compound (II) is prepared in which R' is a hydrogen, in order to obtain, at the end of the decomposition, water as decomposition by-product.

3. Process according to either of Claims 1 and 2, **characterized in that** a compound (I) is prepared in which R₁ is an amino acid residue and R₂ is a hydrogen or a methyl, ethyl or propyl group.

4. Process according to one of Claims 1 to 3, **characterized in that** the decomposition of compound (II) is performed in an anhydrous organic solvent, and the addition reaction consists in adding to the reaction medium an amino acid or a peptide prepared beforehand.

5. Process according to one of Claims 1 to 3, **characterized in that** the decomposition of compound (II) is performed in an aqueous-organic medium with a pH that is substantially between 6 and 10, and the addition reaction consists in adding to the reaction medium an amino acid or a peptide prepared beforehand.

6. Process according to Claim 5, **characterized in that** an aqueous-organic medium buffered by means of an alkaline bicarbonate to a pH of between 6.5 and 7.5 is used to perform the decomposition and the addition, in order to add a single amino acid (originating from the decomposition) to the amino acid or the peptide introduced into the medium.

7. Process according to one of Claims 1 to 3, **characterized in that** the decomposition of compound (II) is performed in an aqueous-organic medium with a pH that is substantially between 6 and 10, and the addition reaction is performed between compound (I) and an amino acid derived from a partial hydrolysis of compound (I).

8. Process according to one of Claims 1 to 7, in which
(a) N- [N'-nitroso-N'-(R')carbamoyl]amino acid is synthesized directly in the reaction medium by the action of a nitrosating agent on an N-[N'-(R')carbamoyl]amino acid of formula:

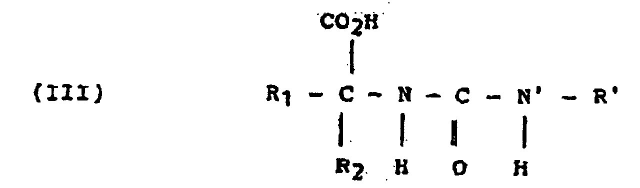

9. Process according to one of Claims 2 to 8, in which N-[N'-nitroso-N'-(R')carbamoyl]amino acid is synthesized directly in the reaction medium in two steps:
• by first reacting isocyanic acid, a salt of this acid or a methyl or chloroethyl isocyanate with an amino acid to obtain an N-[N'-(R')carbamoyl]amino acid of formula: 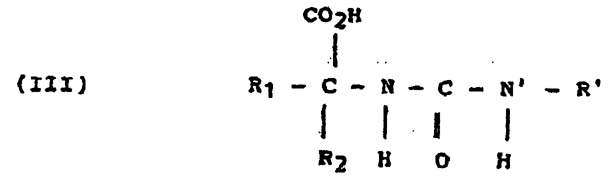 and by then reacting a nitrosating agent with the N-[N'-(R')carbamoyl]amino acid formed.

## Patentansprüche

1. Verfahren zur Synthese von Peptiden, **dadurch gekennzeichnet, dass**
a) eine N-Carboxyanhydrid-Aminosäure mit der Formel 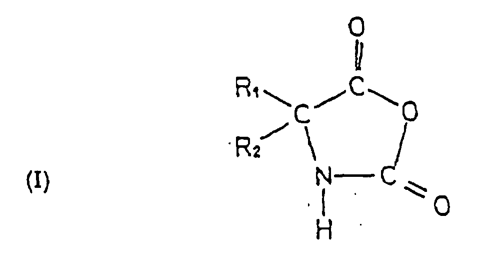 , wobei es sich bei R₁ und R₂ um einen Wasserstoff-Rest oder eine radikallsche Alkyl-Gruppe, Cycloalkyl-Gruppe, Aryl-Gruppe oder Aryl-Alkyl-Gruppe handelt, die entweder nicht, oder mit einer oder mehreren Alkohol-, Thiol- Amin-, Sulfid-, Säure- oder Amidfunktionen substituiert ist, durch Zersetzung von N-[N'-Nitroso-N'-(R')Carbamoyl]-Aminosäure der Formel 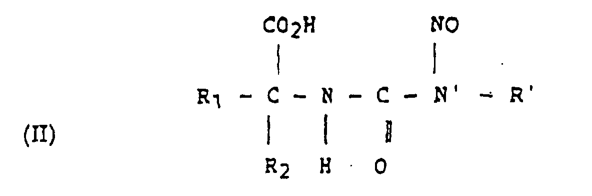 in N₂, R'OH und N-Carboxyanhydrid-Aminosäure hergestellt wird, wobei es sich bei R' um ein Radikal aus der Gruppe H, CH₃, CH₂CH₂Cl handelt;
b) eine Additionsreaktion durchgeführt wird zwischen der Verbindung (I) und einer Aminogruppen enthaltenden Säure oder einem Peptid, das mindestens eine freie α-Aminogruppe aufweist, um ein Dipeptid oder ein Peptid mit einer höheren Wertigkeit in Bezug auf das zugesetzte Peptid zu erhalten.

2. Verfahren gemäß Anspruch 1, **dadurch gekennzeichnet, dass** die Verbindung (II), in der R' ein Wasserstoff-Rest ist, so hergestellt wird, dass man bei der Zersetzung Wasser als Nebenprodukt der Zersetzung erhält.

3. Verfahren gemäß einem der Ansprüche 1 bis 2, **dadurch gekennzeichnet, dass** eine Verbindung (I) hergestellt wird, in der R₁ der Rest einer Amino-Gruppen enthaltenden Säure und R₂ ein Wasserstoff-Rest oder eine Methyl-, Äthyl oder Propyl-Gruppe ist.

4. Verfahren gemäß einem der Ansprüche 1 bis 3, **dadurch gekennzeichnet, dass** die Zersetzung der Verbindung (II) in einem wasserfreien organischen Lösungsmittel stattfindet und die Additionsreaktion auf der Zugabe einer Amino-Gruppen enthaltenden Säure oder eines Peptids, die jeweils vorher hergestellt wurden, zum Reaktionsmedium beruht.

5. Verfahren gemäß einem der Ansprüche 1 bis 3, **dadurch gekennzeichnet, dass** die Zersetzung der Verbindung (II) in einem wässerigen organischen Milieu bei einer Einstellung des pH-Bereichs zwischen 6 und 10 stattfindet und die Additionsreaktion auf der Zugabe einer Amino-Gruppe enthaltenden Säure oder eines Peptids, die jeweils vorher hergestellt wurden, zum Reaktionsmedium beruht.

6. Verfahren gemäß Anspruch 5, **dadurch gekennzeichnet, dass** man zur Durchführung der Zersetzung und der Addition ein wässeriges organisches Milieu einsetzt, das mit Hilfe von alkalischem Bicarbonat in einem pH-Bereich zwischen 6.5 und 7.5 gepuffert wurde, um der Amino-Gruppen enthaltenden Säure oder dem Peptid, die in das Medium eingebracht wurden, eine einzelne Aminosäure (die aus der Zersetzung stammt) zuzugeben.

7. Verfahren gemäß einem der Ansprüche 1 bis 3, **dadurch gekennzeichnet, dass** die Zersetzung der Verbindung (II) in einem wässerigen organischen Milieu bei einer Einstellung des pH-Bereichs zwischen 6 und 10 stattfindet und die Additionsreaktion zwischen der Verbindung (I) und einer Amino-Gruppen enthaltenden Säure, die aus einer partiellen Hydrolyse der Verbindung (I) hervorgegangen ist, stattfindet.

8. Verfahren gemäß einem der Ansprüche 1 bis 7, wobei (a) die N-[N'-Nitroso-N'-(R')Carbamoyl]-Aminosäure direkt in dem Reaktionsmedium durch die Reaktion eines Nitrierungsmittels mit einer N-[N'-(R')Carbamoyl]-Aminosäure der Formel 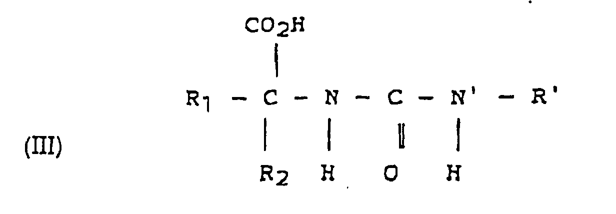 gebildet wird.

9. Verfahren gemäß einem der Ansprüche 2 bis 8, wobei die N-[N'-Nitroso-N'-(R')Carbamoyl]-Aminosäure direkt in dem Reaktionsmedium in zwei Schritten gebildet wird:
• indem man zunächst Isocyansäure, ein Salz dieser Säure oder ein Methyloder Chloräthyl-Isocyanat mit einer Aminosäure reagieren lässt, um eine N-[N'-(R')Carbamoyl]-Aminosäure der Formel: 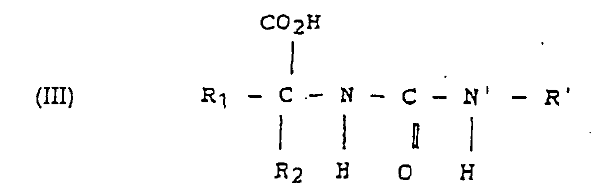 zu erhalten;
• indem man schließlich ein Nitrierungsmittel mit der gebildeten N-[N'- (R')Carbamoyl]-Aminosäure reagieren lässt.
